(19)

**Europäisches Patentamt**
**European Patent Office**
**Office européen des brevets**

(11) **EP 4 631 432 A1**

(12) **EUROPEAN PATENT APPLICATION**
published in accordance with Art. 153(4) EPC

(43) Date of publication:
**15.10.2025  Bulletin 2025/42**

(21) Application number: **22968589.6**

(22) Date of filing: **12.12.2022**

(51) International Patent Classification (IPC):
**A61B 6/00** (2024.01)        **G06T 11/00** (2006.01)

(52) Cooperative Patent Classification (CPC):
**A61B 6/00; G06T 11/00**

(86) International application number:
**PCT/KR2022/020131**

(87) International publication number:
**WO 2024/128328 (20.06.2024 Gazette 2024/25)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **LG Electronics Inc.**
**Yeongdeungpo-gu**
**Seoul 07336 (KR)**

(72) Inventors:
• **KIM, Sunghyun**
**Seoul 06772 (KR)**
• **HONG, Yunpyo**
**Seoul 06772 (KR)**
• **IM, Hyewon**
**Seoul 06772 (KR)**

(74) Representative: **Schott, Jakob Valentin**
**Wuesthoff & Wuesthoff**
**Patentanwälte und Rechtsanwalt PartG mbB**
**Schweigerstraße 2**
**81541 München (DE)**

(54) **IMAGE PROCESSING METHOD, APPARATUS, AND SYSTEM**

(57) An image processing method, apparatus and system are disclosed. Here, the X-ray image processing apparatus according to an embodiment of the present disclosure may comprise: an image analysis unit for analyzing an original X-ray image; a transform unit, which performs multi-scale transform on the original X-ray image on the basis of the analysis result so as to separate same by frequency unit; a tissue processing unit for extracting and normalizing tissue region data on the basis of a residual term corresponding to an X-ray image for a low frequency band from among the separated X-ray images; an inverse transform unit for inversely transforming the X-ray image on the basis of the tissue-normalized residual term; and a control unit for performing tone mapping and output control on the inversely transformed X-ray image.

FIG. 4

Image Analysis (S101)

Log Transform (S103)

Multiscale Transform (S105)

Contrast Control (S107)

Residual Tissue Extraction (S109)

Tissue Plane Estimation (S111)

Background Map Generation (S113)

Tissue Normalization (S115)

Strength Control (S117)

Multiscale Inverse Transform (S119)

Top Mapping (S121)

EP 4 631 432 A1

**Description**

[Technical field]

The present disclosure relates to image processing, and more specifically, to an image processing method, apparatus and system for processing an acquired image so that the contrast is improved.

[Background technology]

As medical devices and associated technologies continue to advance, the medical device market has been undergoing rapid growth. Among various types of medical devices, X-ray medical devices are currently the most widely deployed and are in high demand.

These X-ray medical devices are devices that photograph the inside of the human body for radiation examination.

However, the image acquired from the X-ray medical device, that is, the X-ray image, has an uneven brightness level and is difficult to distinguish by region, making it difficult to make a precise diagnosis or accurate judgment of the X-ray image.

In addition, since X-ray images usually have a wide dynamic range of 16 bits or more and information on the area of interest is densely packed in a narrow area, there was a problem that it was difficult to directly apply the existing general image processing method.

Accordingly, attempts have been made to provide high-quality X-ray images so that more precise diagnosis and accurate judgment may be made on existing X-ray images, but it is still difficult to obtain high-quality X-ray images due to contrast processing problems.

[Detailed Description of the Invention]

[Technical Problem]

The present disclosure has as its first task the provision of an acquired X-ray image by processing it into an image with an appropriate level of balanced contrast for use in diagnosis.

The present disclosure has as its second task the provision of an image by processing it so that the contrast is improved when a difference in contrast occurs between the subject or area of the image being photographed.

The present disclosure has as its second task the provision of an image by processing it so that the contrast is improved due to thickness deviation in the acquired X-ray image.

[Technical Solution]

An X-ray image processing device according to an embodiment of the present disclosure may include an image analysis module configured to analyze an original X-ray image; a transformation module configured to perform a multi-scale transform on the original X-ray image based on the result of the analysis and to separate the image into frequency components; a tissue processing module configured to extract and normalize tissue region data based on a residual term corresponding to a low-frequency band X-ray image among the separated X-ray images; an inverse transformation module configured to reconstruct the **X-**ray image based on the residual term that has undergone tissue normalization; and a control module configured to perform tone mapping and output control of the reconstructed X-ray image.

An X-ray image processing method according to an embodiment of the present disclosure may include analyzing an original **X-**ray image after receiving; performing a multi-scale transform on the original X-ray image based on the analysis result to separate it into frequency components; extracting and normalizing tissue region data based on a residual term corresponding to a low-frequency band X-ray image among the separated images; reconstructing the X-ray image based on the residual term that has undergone tissue normalization; and tone mapping and outputting the reconstructed X-ray image.

An X-ray image processing system according to an embodiment of the present disclosure may include an image acquisition device configured to acquire an X-ray image of a human body; a display; and an image processing apparatus including: an image analysis module configured to analyze an original X-ray image; a transformation module configured to perform a multi-scale transform on the original X-ray image based on the result of the analysis and to separate the image into frequency components; a tissue processing module configured to extract and normalize tissue region data based on a residual term corresponding to a low-frequency band X-ray image among the separated X-ray images; an inverse transformation module configured to reconstruct the X-ray image based on the residual term that has undergone tissue normalization; and a control module configured to perform tone mapping and output control of the reconstructed X-ray image.

[Effect of the invention]

According to at least one of the various embodiments of the present disclosure, there is an effect of improving the brightness contrast of an X-ray image through tissue normalization.

[Brief description of the drawings]

FIG. 1 is a schematic diagram of an image processing system according to an embodiment of the present disclosure.
FIG. 2 illustrates an example of an image processing system of FIG. 1 actually implemented.
FIG. 3 is a block diagram of an image processing device according to an embodiment of the present invention.
FIG. 4 is a flowchart illustrating an image processing method according to an embodiment of the present invention.
FIG. 5 is a diagram illustrating an image analysis method according to an embodiment of the present disclosure.
FIG. 6 is a diagram illustrating a residual image tissue extraction method according to an embodiment of the present disclosure.
FIG. 7 is a drawing for explaining a tissue play estimation (or prediction) method according to an embodiment of the present disclosure.
FIG. 8 is a drawing for explaining a background map creation method according to an embodiment of the present disclosure.
FIGS. 9 to 13 are drawings for explaining a tissue normalization method according to an embodiment of the present disclosure.
FIG. 14 is a drawing for explaining a multi-scale inverse transformation method according to an embodiment of the present disclosure.
FIG. 15 is a drawing for explaining a process of deriving a result value through tissue normalization from a residual image according to an embodiment of the present disclosure.
FIGS. 16 to 19 are drawings for comparing an original image with a result image processed according to an image processing method according to the present disclosure.

[Best Mode]

Hereinafter, embodiments related to the present invention will be described in more detail with reference to the drawings. The suffixes "module" and "part" for components used in the following description are given or used interchangeably only for the convenience of writing the specification, and do not have distinct meanings or roles in themselves.

Image processing methods and devices according to various embodiments of the present disclosure are disclosed.

The "image" described in this specification is described with reference to radiation, particularly an X-ray image obtained from an X-ray device, but is not limited thereto.

Hereinafter, with reference to the attached drawings, the image processing method according to the present disclosure, particularly a method and device for processing an image so that contrast according to thickness deviation in an image in a low-frequency band is improved, will be mainly described.

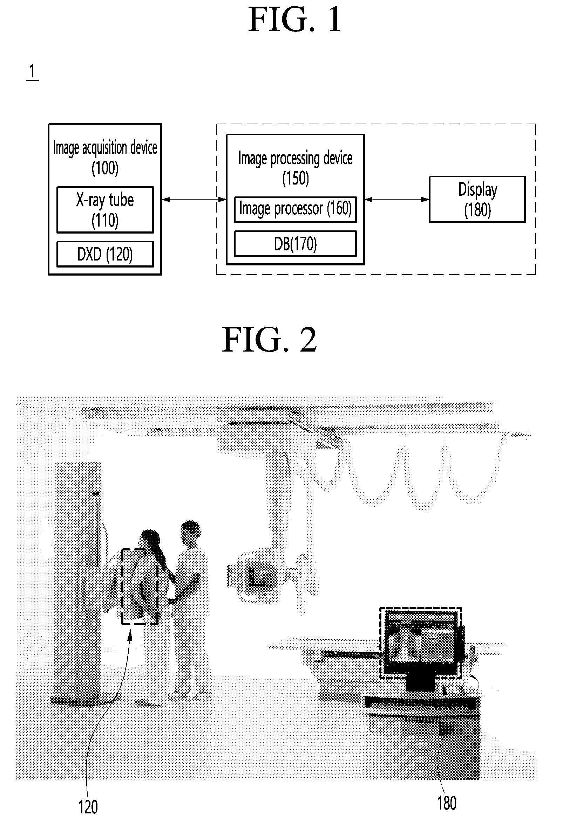

FIG. 1 is a schematic diagram of an image processing system 1 according to one embodiment of the present invention.

FIG. 2 illustrates an example of an image processing system 1 of FIG. 1 actually implemented.

The image processing system 1 may largely include an image acquisition device 100, an image processing device 150, and an image output device 180.

In FIG. 1, the image processing device 150 and the image output device 180 are expressed as a set configuration, but are not limited thereto. For example, the image output device 180 may be implemented as a component independent of the image processing device 150. For example, the image output device 180 may be a terminal of a medical institution.

Referring to FIGS. 1 and 2, the image processing system 1 is described as follows.

The image acquisition device 100 may include an X-ray tube 110 and a digital X-ray detector 120.

The X-ray tube 110 irradiates a certain amount of X-ray to a subject to be photographed (e.g., a body part).

The digital X-ray detector 120 detects an X-ray image of the subject to be photographed based on the X-ray irradiated through the X-ray tube 110.

The image acquisition device 100 may transmit the detected image to the image processing device 150.

The image acquisition device 100 and the image processing device 150 (which may also include an image output device 180) may perform data communication to exchange data with each other through a wired/wireless network.

The image processing device 150 may process the received X-ray image and provide it to the user through the image output device, i.e., the display 180.

According to one embodiment, the image processing device 150 is a computing device and may be a remotely located server device. Therefore, the image processing device 150 may be described by other names such as a computing device, a server, a controller, etc.

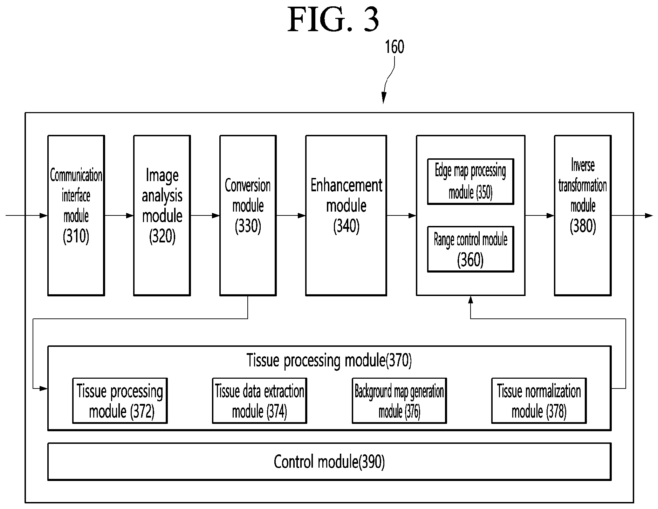

FIG. 3 is a block diagram of the image processing device 150 according to one embodiment of the present invention.

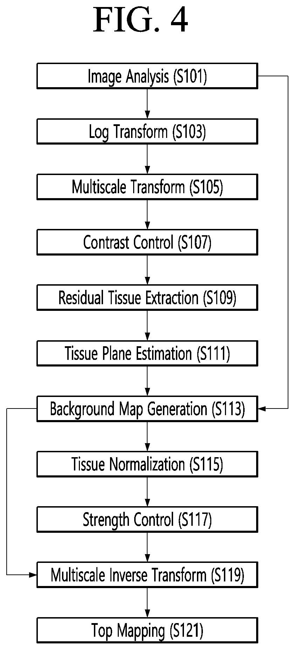

FIG. 4 is a flowchart illustrating an image processing method according to one embodiment of the present invention.

Referring to FIG. 1, the image processing device 150 may be configured to include an image processor 160 and a DB 170.

Referring to FIG. 3, the image processor 160 may be implemented by including a communication interface module 310, an image analysis module 320, a conversion module 330, an enhancement module 340, a first processing module, a tissue processing module 370, an inverse conversion module 380, a control module 390, etc. The first processing module may include an edge map processing module 350, a range processing module 360, etc.

Depending on the embodiment, some of the components illustrated in FIG. 3 may be modularized or vice versa. According to another embodiment, at least one or more components may be added to FIG. **3.**

Each component of the image processor 160 illustrated in FIG. 3 is described together with FIG. 4.

The image processing method of FIG. 4 is described from the perspective of the image processing device 150 or the image processor 160, but is not limited thereto.

In step S101, the communication interface module 310 may receive an original or raw X-ray image from, for example, the image acquisition device 100, and the image analysis module 320 may analyze the received original X-ray image.

In step S103, the transformation module 330 may transform the original X-ray image in the first order. Here, the first transformation may be, for example, a log transformation, but is not limited thereto.

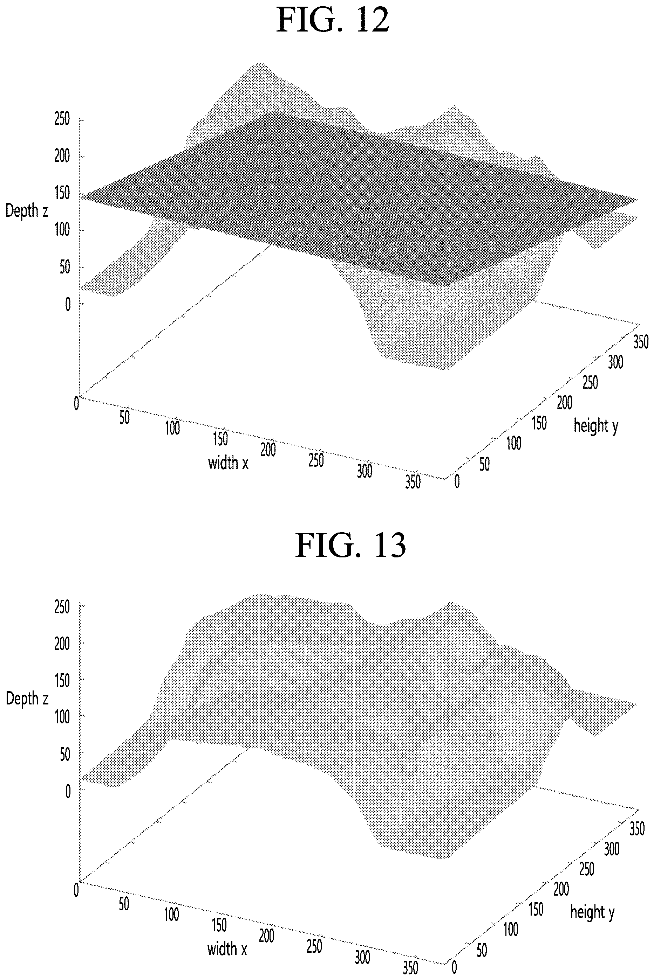

In step S105, the transformation module 330 may transform the first-transformed X-ray image in the second order. Here, the second transformation may be, for example, a multi-scale transformation that separates the X-ray image into n layers (where n is a natural number greater than or equal to 2) in frequency units, as illustrated in FIG. 12, but is not limited thereto.

In step S107, the image processing device 150 may perform contrast control on the second-converted X-ray image. Here, the contrast control may be performed, in particular, on a high-frequency layer among the multiple layers, i.e., a high-frequency band.

Below, the method of improving contrast by processing thickness deviation in the low-frequency band, which is separated by second-converting an X-ray image through step S105 in an image processing device 150, is described.

In the present disclosure, processing for the residual term, as shown in FIG. 12, among the low-frequency bands, is described as an example, but is not limited thereto.

Meanwhile, below, processing for thickness deviation in the residual term in the low-frequency band, as described above, is described as an example, particularly processing a tissue portion.

In relation to this, processing for the tissue portion may be processed by a tissue processing module 370.

Referring to FIG. 3, the tissue processing module 370 may be configured to include a tissue data extraction module 372, a tissue plane estimation module 374, a background map generation module 376, and a tissue normalization module 378, but is not necessarily limited thereto.

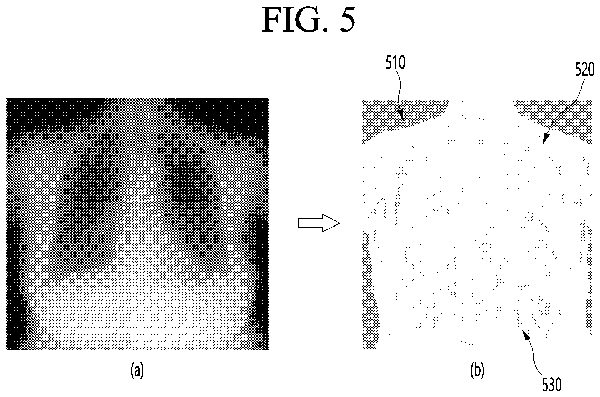

In step S109, the tissue data extraction module 372 may extract data corresponding to a tissue candidate region 530 that may be extracted as a segmentation result in the image analysis process from a residual term of a low frequency band, as shown in (b) of FIG. 5. At this time, the extracted data may be named as residual tissue or residual tissue data, etc.

In step S111, the tissue plane estimation module (or prediction module) 374 may estimate or predict the tissue plane (or tissue model plane) based on the residual tissue data extracted in step S109.

In step S115, the tissue normalization module 378 may perform tissue normalization based on the tissue plane estimated or predicted in step S111.

Meanwhile, the tissue normalization module 378 may utilize a background map in the process of performing tissue normalization in step S115.

In step S113, the background map generation module 376 may generate a background map.

Since the background area is an area outside the normalization range, a weight map may be generated. The background map may be formed by referring to the background area.

In other words, the tissue normalization module 378 may perform tissue normalization based on the tissue plane estimated or predicted in step S111 and the background map generated in step S113 in step S115.

In step S117, the image processing device 150 may perform strength control on the tissue normalized X-ray image.

Steps S109 to S117 performed in the image processing device 150 may represent a processing method for improving the low-frequency band according to the present disclosure.

In step S119, the image processing device 150 may perform a multi-scale inverse transform to correct the low-frequency band signal and the high-frequency band signal improved through the above-described process.

Here, Laplacian weighting correction may be used for high-frequency band compensation in the inverse transformation process. However, it is not limited thereto.

In step S121, the image processing device 150 may control the display 180 to output after tone mapping when the multi-scale inverse transformation process for the X-ray image signal is completed through step S119.

Referring to FIG. **3,** the communication interface module 310 provides an interfacing environment for data communication with the image acquisition device 100 and may receive an X-ray image from the image acquisition device 100.

The communication interface module 310 may perform short range communication with the image acquisition device 100. To this end, the communication interface module 310 may support short-range communication using at least one of Bluetooth™, BLE (Bluetooth Low Energy), RFID (Radio Frequency Identification), Infrared Data Association (IrDA), UWB (Ultra Wideband), ZigBee (ZigBee), NFC (Near Field Communication), Wi-Fi (Wireless-Fidelity), Wi-Fi Direct, and Wireless USB (Wireless Universal Serial Bus) technologies. The communication interface module 310 may support wireless communication between the image acquisition device 100 and the wireless communication system, between the image processing device 150 and the display 180, or between the image processing device 150 and the network where the server is located through short-range wireless communication networks (Wireless Area Networks). However, the present disclosure is not limited thereto, and wired communication or other wireless communication methods may be used in addition to the aforementioned short-range communication.

The image analysis module 320 may perform analysis on the received X-ray image.

FIG. 5 is a drawing for explaining an image analysis method according to an embodiment of the present disclosure.

An image processing device 150 may segment background, anatomy, collimation, metal object, etc. from a received original X-ray image and store the information.

(a) of FIG. 5 illustrates a received original X-ray image, and (b) of FIG. 5 is a drawing for explaining the analysis result of the image shown in (a) of FIG. 5.

An image analysis module 320 may generate and provide analysis result data such as that shown in (b) of FIG. 5 from the original image data shown in (a) of FIG. 5.

Referring to (b) of FIG. 5, the analyzed X-ray image may include a direct exposure region 510 and an anatomy region 520.

Meanwhile, a tissue region (tissue area) according to the present disclosure may be defined, for example, from an anatomy region 520. For example, a region that is flat and has a size greater than a predetermined threshold in the extracted anatomy region 520 may be defined as a tissue region (for example, part 530 in (b) of FIG. 5). However, (b) of FIG. 5 is an image analysis step, and the tissue region at this time may be named a tissue candidate region 530 rather than a confirmed tissue region.

That is, the image analysis module 320 may predict each region shown in (b) of FIG. 5 and generate a map using the segmentation technology.

In the present disclosure, the segmentation technology based on an object base or a histogram base is used as an example, but is not limited thereto.

Meanwhile, segmentation of the body, background region, etc. may be classified using various general segmentation algorithms, such as region growing, Otsu's threshold, mean shift, K means clustering, etc.

In addition, as shown in (b) of FIG. 5, a flat region inside the body after segmentation may also be obtained using various feature information. In relation to the above feature information, detection methods such as canny edge, Laplacian edge, Sobel, Prewitt, and Roberts edge may be used.

The image analysis module 320 may predict the tissue candidate region 530 and generate a map by applying the segmentation technique as shown in (b) of the original X-ray image as shown in (a) of FIG. 5, and then enhance the desired region (e.g., the edge region for the tissue candidate region 530) through the enhancement module 340.

Meanwhile, according to one embodiment of the present disclosure, in the image segmentation process described above, it is not necessary to extract the tissue candidate region exactly along the shape.

Since one embodiment of the present disclosure is a method of predicting a 3D linear plane through regression by inputting a certain number or more of candidate data samples, it is sufficient to extract only a certain signal.

In relation to this, in the present disclosure, a flat surface without an edge is selected within an already segmented body region, and if the number of selected surfaces is less than a threshold, a signal around a body line may be additionally extracted. Meanwhile, body segmentation may utilize an area segmentation method.

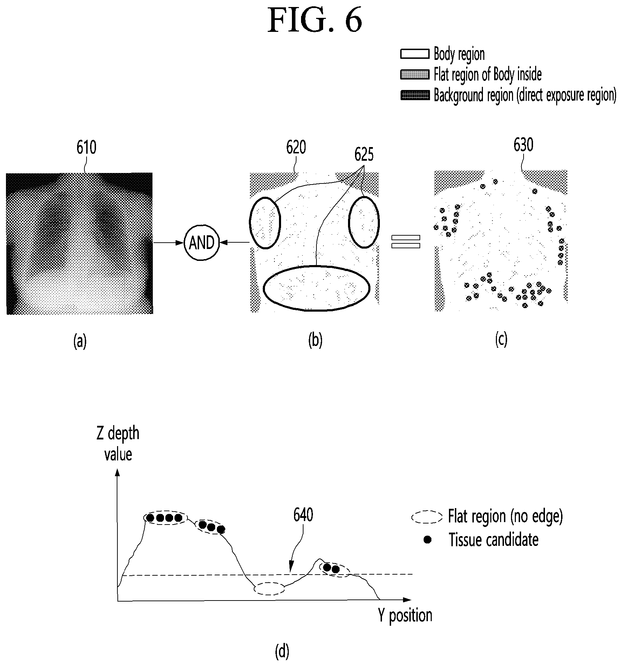

FIG. 6 is a drawing illustrating a method for extracting a residual image tissue according to an embodiment of the present disclosure.

FIG. 6 particularly describes extracting tissue data from a residual term in a low-frequency band.

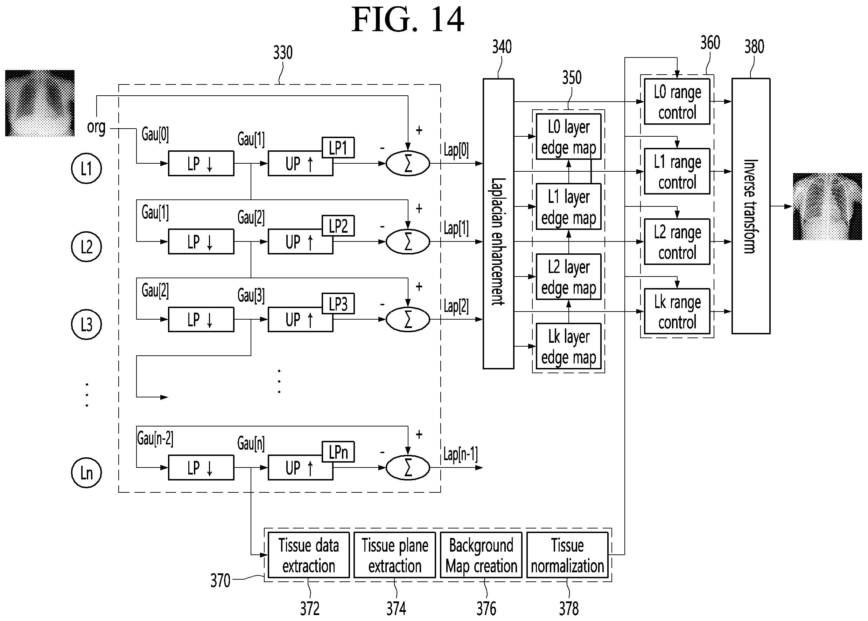

In the present disclosure, in order to determine the brightness deviation of the entire X-ray image, the last residual term of the image pyramid illustrated in FIG. 14, which has almost no high-frequency edge signal, may be selected and utilized.

According to another embodiment, not only the last image pyramid but also at least one or more image pyramids before it may be selected and utilized.

The image analysis module 320 may generate a ROI (Region of Interest) map.

The tissue data extraction module 372 may extract a residual term signal corresponding to the tissue data location after resizing the ROI map according to the residual term resolution. The residual term may use a technology that adjusts the overall brightness latitude.

Referring to FIG. 6, the tissue data 630 may be extracted based on the residual term 610 illustrated in (a) of FIG. 6 and the ROI map 620 illustrated in (b) of FIG. 6.

The circle 625 in the ROI map 620 illustrated in (b) of FIG. 6 may represent a tissue candidate area.

Meanwhile, (d) of FIG. 6 is a graph (horizontal axis represents Y position and vertical axis represents Z depth value) drawn to explain a flat region without an edge and a tissue candidate based on a brightness average value (e.g., flat region average threshold) 640.

Referring to (d) of FIG. 6, in the present disclosure, if air is included in a flat region such as a lung, it is relatively dark, so the corresponding data should be excluded so that the tissue region may be well expressed, and therefore only data that is higher than the average of the body region may be selected. In relation to the graph of (d) of FIG. 6 and the data selection, the following mathematical expressions 1 and 2 may be used. However, the present disclosure is not limited thereto.

【Mathematical Formula 1】

$$Flat\ region\ avg = \frac{\sum Flat\ region\ depth\ (x,y)}{N}$$

【Mathematical Formula 2】

$$Tissue\ data\ set\ (x,y) = \{Flat\ data\ set\ (x,y) > Flat\ region\ avg\}$$

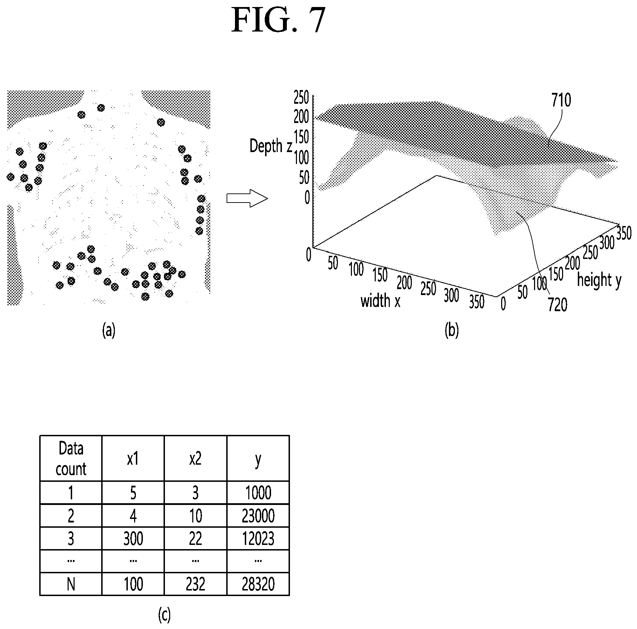

FIG. 7 is a diagram illustrating a tissue plane estimation (or prediction) method according to an embodiment of the present disclosure.

FIG. 7 discloses a method for predicting a reference plane representing tissue depth based on tissue candidate data. In this case, the present disclosure uses a linear data regression method as an example for predicting the reference plane, but is not limited thereto.

(a) of FIG. 7 illustrates tissue data illustrated in (c) of FIG. 6 described above, and (b) of FIG. 7 illustrates a tissue data set (1 - N) based on the tissue data illustrated in (a) of FIG. 7.

In (b) of FIG. 7, x1 may represent the x-axis coordinate of the image, x2 may represent the y-axis coordinate of the image, and y may represent the brightness value (depth) of the image.

In (c) of FIG. 7, a tissue plane estimation graph based on (a) and (b) of FIG. 7 is shown.

The tissue plane estimation graph may be expressed as a 3D plot, and includes a residual term 710 and a tissue plane 720.

Referring to (b) of FIG. 7, the 3D linear plane equation may refer to, for example, the mathematical expression 3 below, but is not limited thereto.

【Mathematical expression 3】

$$Y = a + b1X1 + b2X2$$

Meanwhile, with respect to (c) of FIG. 7, the calculation (linear regression) for the coefficients of the 3D linear plane equation may be modeled as a non-linear equation, if necessary. Meanwhile, the method of finding the solution may utilize, for example, the regression method or SVD (Singular Value Decomposition), and the following mathematical expressions 4 to 6 may be referred to. However, it is not limited thereto.

**【Mathematical Formula 4】**

$$a = \frac{\sum y}{N} - b1 \frac{\sum x1}{N} - b2 \frac{\sum x2}{N}$$

**【Mathematical Formula 5】**

$$b1 = \frac{(\sum x2^2)(\sum x1y) - (\sum x1x2)(\sum x2y)}{(\sum x1^2)(\sum x2^2) - (\sum x1x2)^2}$$

**【Mathematical Formula 6】**

$$b2 = \frac{(\sum x1^2)(\sum x2y) - (\sum x1x2)(\sum x1y)}{(\sum x1^2)(\sum x2^2) - (\sum x1x2)^2}$$

The tissue plane estimation module 374 may generate a plane by modeling tissue depth according to location in three dimensions based on such characteristics, for example, the degree of thickness or thinness of tissue in an X-ray signal may have weak diagnostic significance.

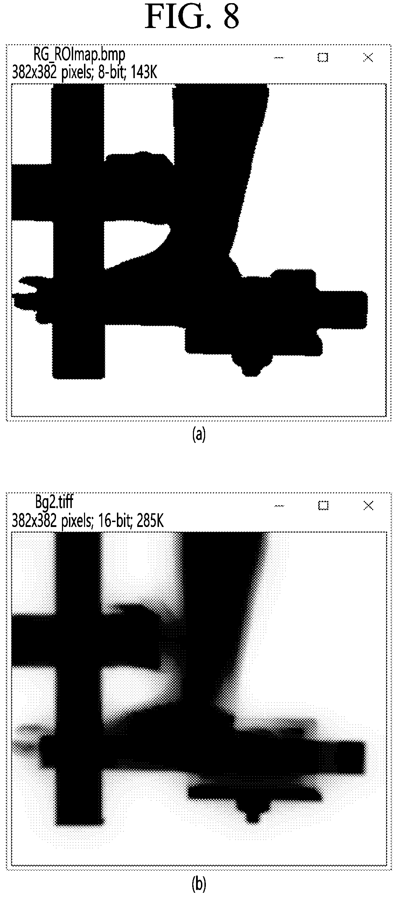

FIG. 8 is a drawing illustrating a method for creating a background map according to an embodiment of the present disclosure.

When tissue normalization is applied to a part other than the body area, the background area may also be affected. This may cause the background area to be brighter than the body area during a subsequent tone mapping process. Therefore, separation of the background area is necessary so that the background area is not normalized.

The background map generation module 376 may generate a map for the background area other than the body area, that is, a background map, in order to identify and separate the background area.

In order to create the background map, the present disclosure uses an alpha matting technique as an example to accurately create a gray background map, but is not limited thereto.

In relation to this, (a) of FIG. 8 represents a binary background map, and (b) of FIG. 8 represents a gray background map.

The background map generation module 376 may generate a weight map so that the background area may be reflected to a minimum in order to prevent unnecessary background changes and image distortion in the tone correction step when all areas are normalized with tissue plane information.

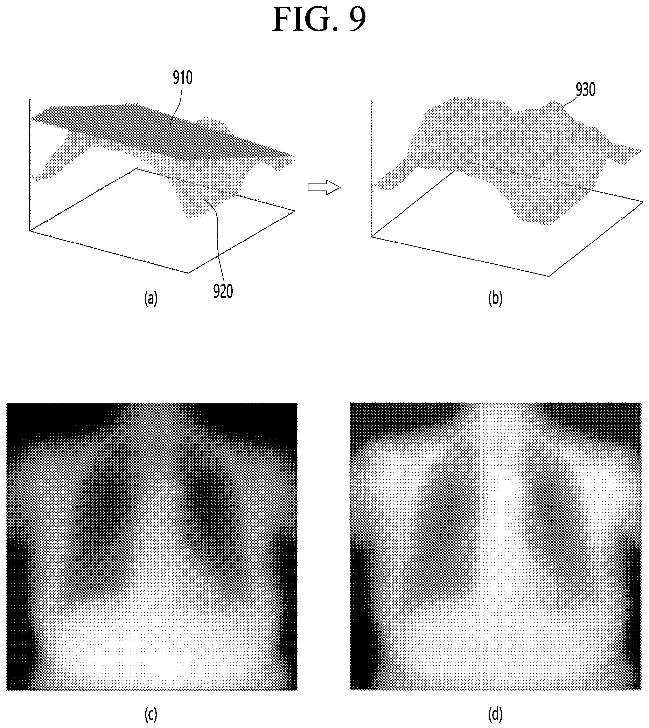

FIGS. 9 to 13 are drawings illustrating a tissue normalization method according to an embodiment of the present disclosure.

FIG. 9 discloses a method for normalizing a residual term based on tissue normalization, that is, a tissue plane predicted in (c) of FIG. 7.

(a) of FIG. 9 illustrates a tissue plane screen (a 3D plot of the residual term and the tissue plane) predicted in (c) of FIG. 7, and (b) of FIG. 9 illustrates a 3D plot of the residual term normalized based on (a) of FIG. 9.

It may be seen that the X-ray image of (c) of FIG. 9 before normalization is improved by being normalized as in (d) of FIG. 9.

That is, the tissue normalization module 378 may normalize the residual term image to a certain depth value using the estimated tissue plane and the background map. In this way, the contrast of the signals of the major diagnostic areas may be processed so that they are consistent. At this time, the following mathematical expression 7 may be referred to.

**【Mathematical Formula 7】**

$$\left( \frac{Norm\ value\ (fixed)}{Estimated\ Depth\ value\ (x,y)} \right) \times input\ residual\ (x,y)$$

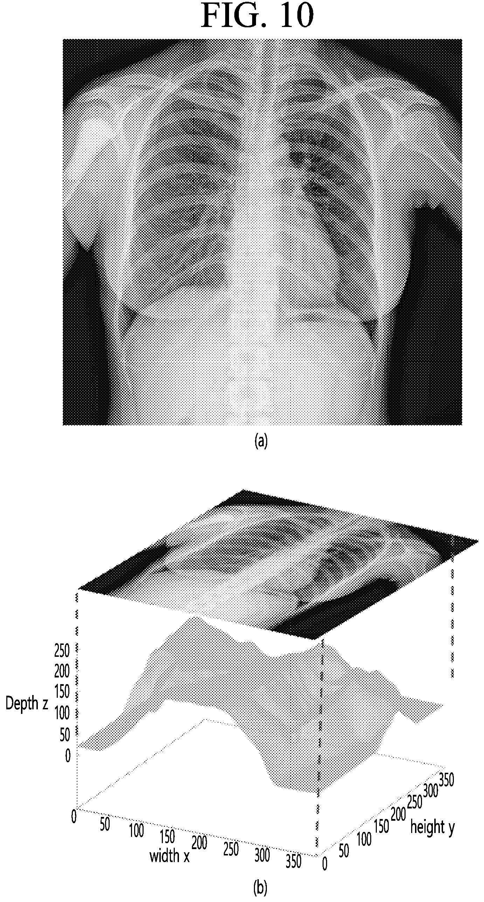

(a) of FIG. 10 illustrates an X-ray image, and (b) of FIG. 10 illustrates the generation of a 3D plot for the X-ray image.

Since the X-ray image may be viewed as a depth signal for thickness, when expressed in 3D dimension, the degree of thickness may be expressed as the z-axis.

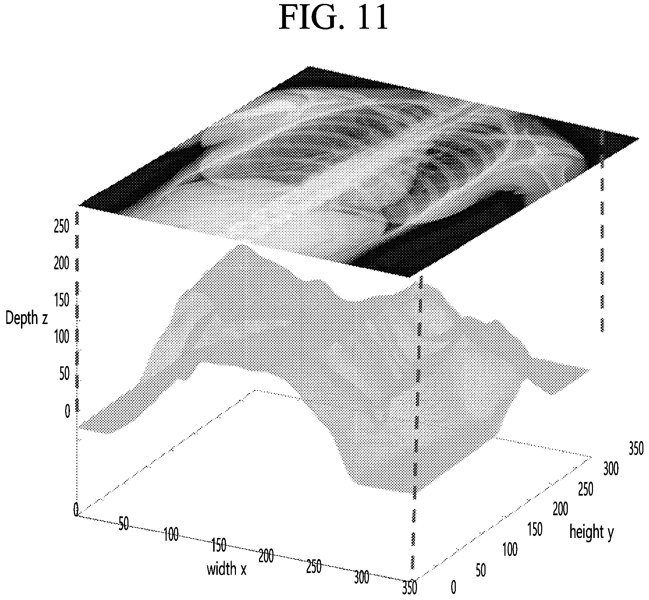

Meanwhile, FIGS. 11 to 13 show a tissue normalization processor, which includes tissue depth estimation and normalization.

FIG. 11 illustrates a 3D plot for an original low-frequency X-ray image, FIG. 12 illustrates estimation of the base tissue depth plane of FIG. 11, and FIG. 13 illustrates the result of normalizing the tissue depth.

FIG. 14 is a drawing illustrating a multi-scale inverse transform method according to an embodiment of the present disclosure.

After tissue normalization for low-frequency signals is performed, they may be combined with high-frequency signals. However, in this case, since normalizing depth brightness based on tissue may make high-frequency signals that were hidden inside stand out, it may be necessary to perform compensation to appropriately attenuate high-frequency signals in the inverse transform stage to prevent high-frequency signals from being excessively prominent.

As a method for compensating high-frequency signals, the present disclosure uses a Laplacian weighting compensation method as an example. However, it is not limited thereto. For example, the Laplacian weighting compensation method may be performed so that the final image may be consistent by adjusting high-frequency signals lower as the degree of normalization becomes stronger.

Referring to FIG. 14, the image processing device 150 may obtain an image suitable for a target area, for example, by dividing into frequency units based on multi-scale (or multi-frequency) and adjusting parameters by frequency. As described above, the present disclosure uses a Gaussian-Laplacian pyramid that may be used as a multi-scale conversion (or multi-frequency conversion) method, but is not limited thereto.

The conversion module 330 may perform multi-scale conversion so that the original X-ray image data (or raw X-ray image data) received through the communication interface module 310 may be enhanced by characteristics of the frequency unit, as described above.

Referring to FIG. 14, the conversion module 330 may form n layers (where n is a natural number) based on the original X-ray image, that is, a multi-layer structure.

The above n may be determined according to the setting of the image processing device 150, and for example, if n is 10, a total of 10, i.e., L1 - L10, may be processed.

At this time, the smaller n is, the closer it is to the original X-ray image, and thus, more noise may be included compared to the case where n is relatively large. Therefore, in the present disclosure, Gaussian information corresponding to brightness in layers corresponding to the high-frequency region, i.e., L1 to L3, may be utilized for noise prediction (or estimation).

The enhancement module 340 may enhance the Laplacian value output in layers.

According to an embodiment, the enhancement module 340 may include an edge map processing module 350, a range control module 360, a contrast processing module (not shown), etc.

According to an embodiment, the conversion module 330 and the enhancement module 340 may be modularized.

The edge map processing module 350 may generate an edge map based on the analysis result of the original X-ray image.

The range control module 360 may perform range control for each layer of the multi-scale converted X-ray image based on the analysis result of the original X-ray image. Here, the range control refers to, for example, something related to the consistency of image quality, and may be viewed as an operation that enables consistent image quality processing regardless of the dose by contrast normalization in a certain range(s) where a result is derived using a reference value. This range control may be performed by adjusting the standard deviation for each layer and each image. Meanwhile, the reference value in the above indicates a reference value according to the characteristics of each layer, and the reference value may be different for each layer. In addition, these reference values may be applied to values previously calculated through experiments, etc.

The contrast processing module (not shown) may control and process the contrast of a high-frequency region. The contrast processing module may perform detail contrast enhancement through high-frequency layer contrast control based on the analysis results of the original X-ray image.

In relation to the present disclosure, points requiring processing or improvement may all be different depending on the characteristics of the X-ray image, such as each target area and each target region.

The range control module 360 may perform range control for each layer of the second converted X-ray image based on the analysis results of the original X-ray image.

The image processing device 150 may form a multi-featured-based edge map. In this case, multi-featured may collectively mean information about edges, contrasts, layers, etc., but is not limited thereto.

The edge map processing module 350 may predict the noise level of each Laplacian by using Gaussian information corresponding to brightness, anatomy, layer-by-layer information, etc. in the high-frequency layers (Layer 0 to 3, 4).

Referring to FIG. 14, the present disclosure may calculate the local standard deviation value, but when an edge map is generated using only the calculated local standard deviation value, the edge directional information is not considered, so the edge area may be messy. In addition, in the above case, when the image processing device 150 independently generates a map by layer without considering correlation between layers, the higher the frequency layer, the more limited the noise removal may be.

In the present disclosure, considering this problem, in addition to the calculated local standard deviation value, a more robust map may be generated by reflecting local edge information and correlation between layers of a specific frequency unit.

Referring to FIG. 14, the correlation may be divided based on noise prediction information that combines the edge map and the contrast map and predicts the combined value. This may be, for example, to determine whether to suppress the intensity (strength) of the map according to the noise.

Referring to FIG. 14, the image processing device 150 may perform map-based layer contrast & noise control & local contrast consistency processing.

At this time, the image processing device 150 may perform adaptive Laplacian boost according to the target criterion (Tn) of the Laplacian change rate for each region and each layer defined in advance, as shown in FIG. 14, and range adjustment and edge map.

The image processing device 150 may assign weights to the Laplacian values of each layer using the edge map, as shown in FIG. 14.

The X-ray image has an optimal range (Laplacian) for each shooting region (e.g., chest, hand, pelvis, etc.) and for each frequency unit of the same region (layer 0, 1, 2...). Therefore, the image processing device 150 may collect X-ray images captured with the most optimal dose for each shooting area, and measure the appropriate range of the Laplacian for each layer of the collected X-ray images.

In the present disclosure, for convenience, when measuring the range, instead of measuring in the entire area, only the values for the anatomy area predicted as a result of the image analysis in (b) of FIG. 5 may be selectively performed.

Based on the optimal range values for each area and each layer obtained in advance, when an input image (X-ray image) is received, the range is obtained for each layer and the weight is automatically adjusted according to the reference value, so that images of similar areas may generate consistent image quality.

Regarding the weight, the following mathematical expressions 8 and 9 may be referred to.

$$【Mathematical\ expression\ 8】$$

$$L_{k\_std} = \frac{\sum (L_k(i) - L_{k\_avg})^2}{WH}$$

In mathematical expression 8, Lk_std may represent the standard deviation of the kth Laplacian, and Lk_avg may represent the average value of the kth Laplacian. In addition, WH may represent the product of width and height.

$$【Mathematical\ expression\ 9】$$

$$Cont\_weight_k = a * \exp^{b*(L_k)}$$

In mathematical expression 9, Cont_weight_k may represent a weight value to be multiplied by the kth Laplacian, and a and b may represent weight adjustment parameters. At this time, a is between 5 and 15, b is between -1 and -10, however, a is preferably between 10 and 12 and b is preferably between -2 and -5, but is not limited thereto.

The inverse transformation module 380 may inversely transform the X-ray image processed through the above-described process. At this time, the inverse transformation may be IFFT (Inverse FFT) corresponding to multi-scale transformation.

The image processing device 150 inversely converts the multi-scale converted X-ray image, which may include processing processes such as local contrast consistency, noise reduction, and edge correction.

In addition, the control module 390 may perform a tone mapping operation on the inversed X-ray image to control the output. For example, the tone mapping may indicate that the **X-**ray image itself is a 16-bit image with a wide range, while the display 180 is an 8-bit image, and thus the range does not match, and thus this is corrected.

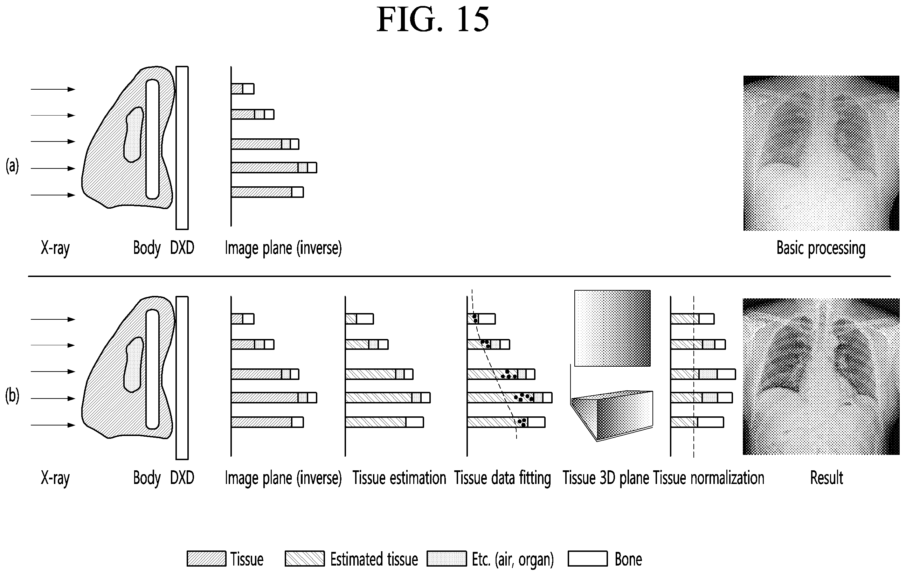

FIG. 15 is a drawing illustrating a process of deriving a result value through tissue normalization from a residual image according to an embodiment of the present disclosure.

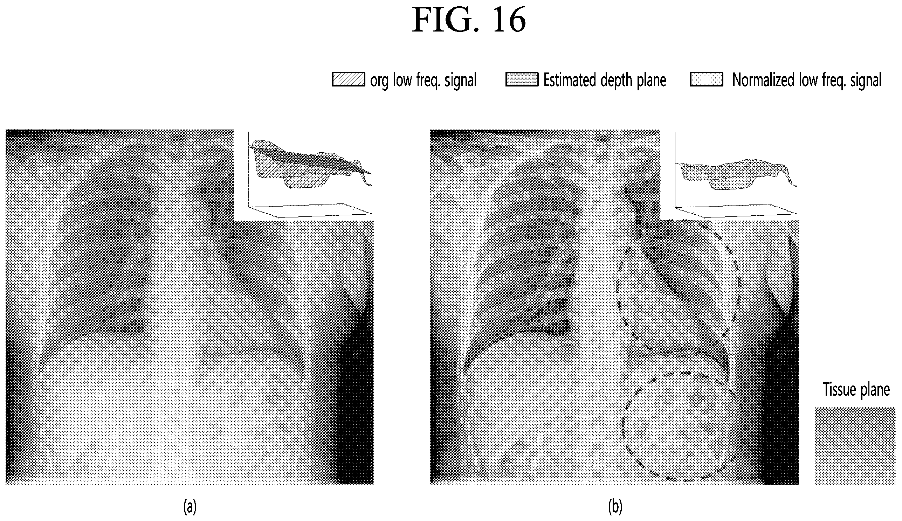

FIGS. 16 to 19 are drawings illustrating a comparison between an original image and a result image processed according to an image processing method according to the present disclosure.

FIG. 15 may be a drawing comparing a tissue plane estimation process from the aforementioned residual image.

(a) of FIG. 15 illustrates a basic processing, that is, an input and a result image to which the present disclosure is not applied, and (b) of FIG. 15 illustrates a result image that is post-processed for the same input X-ray image to which the present disclosure is applied.

In the present disclosure, the residual image of the pyramid may be viewed as a distribution map of an overall X-ray transmission signal, but it may include background, tissue, air, bones, etc.

Therefore, assuming that the tissue component represents the overall X-ray deviation, it is desirable to estimate (linearly) the depth plane based on the tissue component.

And it is necessary to uniformly correct the residual image throughout the image according to the estimated depth plane.

In (b) of FIG. 15, it may be seen that the result value, for example, the contrast, is clearly improved by additionally performing the tissue estimation, tissue data fitting, tissue 3D plane estimation, and tissue normalization processes compared to the basic processing.

FIGS. 16 to 19 illustrate comparison screens of the original low-frequency signal and the low-frequency signal processed according to the present disclosure. In each drawing, the dotted circle may be seen to indicate a particularly improved part.

For example, (a) of FIG. 16 illustrates the original low-frequency signal of the chest area, and (b) of FIG. 16 illustrates the low-frequency image post-processed (i.e., normalized) according to the present disclosure.

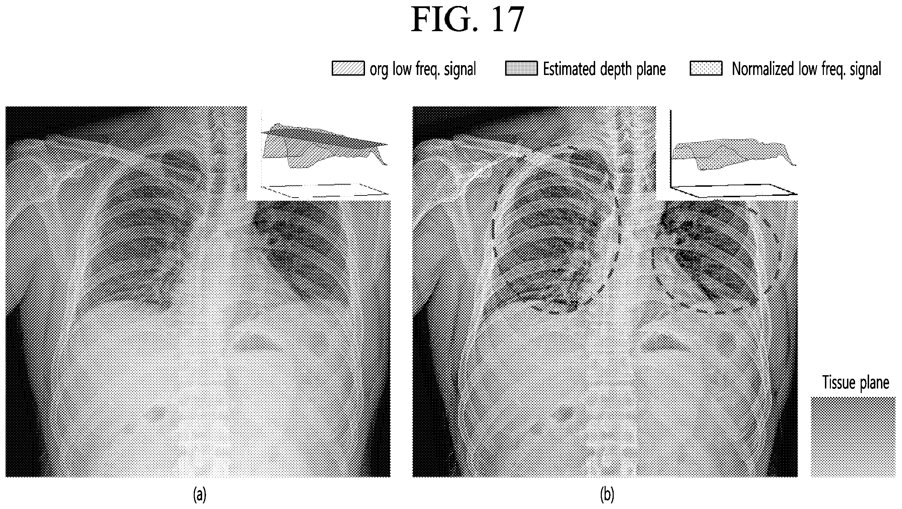

(a) of FIG. 17 illustrates the original low-frequency signal of the rib area, and (b) of FIG. 17 illustrates the low-frequency image post-processed (i.e., normalized) according to the present disclosure.

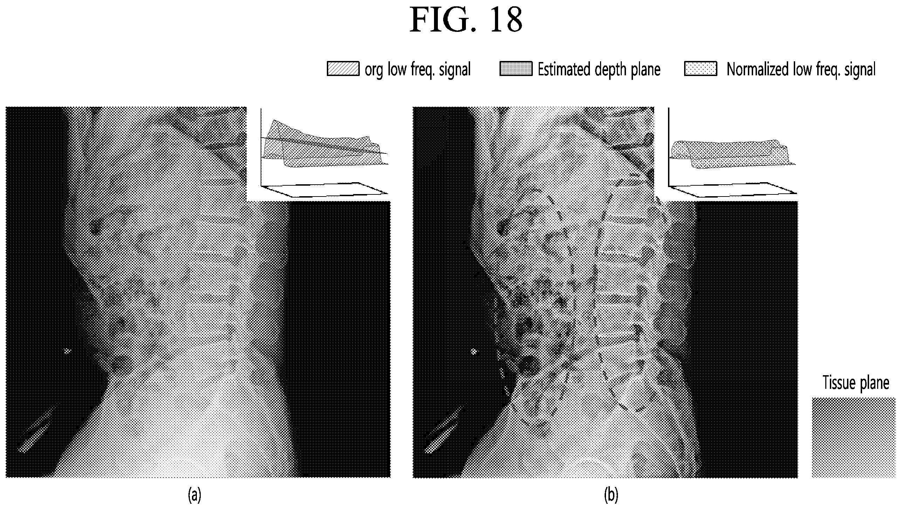

(a) of FIG. 18 illustrates the original low-frequency signal of the L-spine lateral, and (b) of FIG. 18 illustrates the low-frequency image post-processed (i.e., normalized) according to the present disclosure.

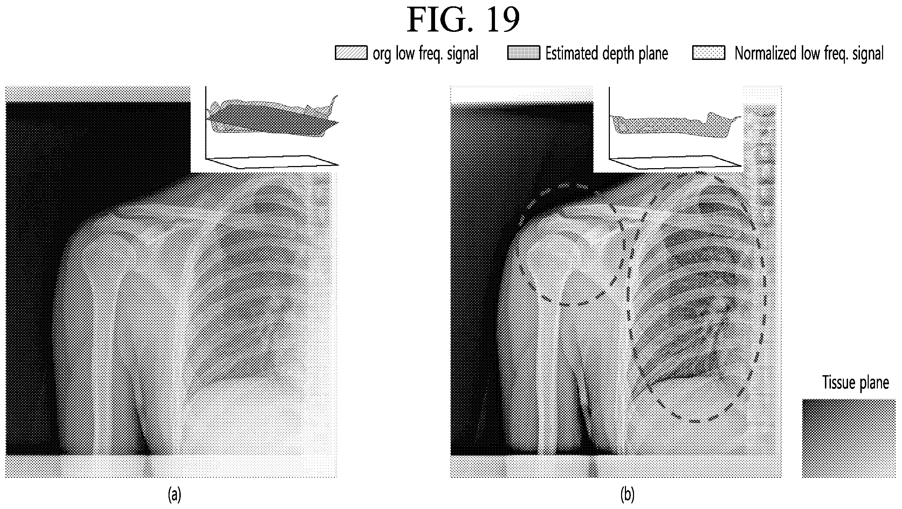

(a) of FIG. 19 illustrates the original low-frequency signal of the shoulder area, and (b) of FIG. 19 illustrates the low-frequency image post-processed (i.e., normalized) according to the present disclosure.

Although the present specification has described using an X-ray image as an example, it is not necessarily limited thereto. In addition, the present invention may be applied in the same or similar manner to various industrial fields that utilize X-rays in addition to the medical field.

The above description is merely an example of the technical idea of the present invention, and those with ordinary knowledge in the technical field to which the present invention belongs may make various modifications and variations without departing from the essential characteristics of the present invention.

Therefore, the embodiments disclosed in the present invention are not intended to limit the technical idea of the present invention but to explain it, and the scope of the technical idea of the present invention is not limited by these embodiments.

The protection scope of the present invention should be interpreted by the claims below, and all technical ideas within the equivalent scope should be interpreted as being included in the scope of the rights of the present invention.

**[Industrial Applicability]**

According to the image processing device according to the present disclosure, the contrast according to the thickness deviation of the image in the low-frequency band is improved, and the brightness deviation may be balanced, so the industrial applicability is remarkable.

**Claims**

1. An image processing device comprising:

   an image analysis module configured to analyze an original X-ray image;
   a transformation module configured to perform a multi-scale transform on the original X-ray image based on the result of the analysis and to separate the image into frequency components;
   a tissue processing module configured to extract and normalize tissue region data based on a residual term corresponding to a low-frequency band X-ray image among the separated X-ray images;
   an inverse transformation module configured to reconstruct the X-ray image based on the residual term that has undergone tissue normalization; and
   a control module configured to perform tone mapping and output control of the reconstructed X-ray image.

2. The image processing device of claim 1, wherein the X-ray image is segmented into a background region, an anatomy region, and a collimation region, wherein the anatomy region includes a tissue candidate region, and the tissue processing module is configured to segment the residual term to extract tissue region data corresponding to the tissue candidate region.

3. The image processing device of claim 2, wherein the image analysis module is configured to extract a tissue candidate region from the anatomy region using a method that determines the tissue candidate region based on regression to predict a 3D linear plane from a predetermined number of tissue candidate data samples.

4. The image processing device of claim 3, wherein the tissue processing module is further configured to predict a tissue model plane based on the extracted tissue region data.

5. The image processing device of claim 4, wherein the tissue processing module is further configured to generate a background map for the background region.

6. The image processing device of claim 5, wherein the tissue processing module is further configured to normalize the residual term based on the predicted tissue model plane information.

7. The image processing device of claim 6, wherein the transformation module uses a Gaussian-Laplacian pyramid structure and further comprises:

an edge map processing module configured to generate edge maps for each layer corresponding to high-frequency components; and
a range control module configured to correlate each layer corresponding to high-frequency components and determine whether to suppress or the suppression strength for the edge maps of each layer, based on tissue normalization-based weighting values.

8. An image processing method comprising:

analyzing an original X-ray image after receiving it;
performing a multi-scale transform on the original X-ray image based on the analysis result to separate it into frequency components;
extracting and normalizing tissue region data based on a residual term corresponding to a low-frequency band X-ray image among the separated images;
reconstructing the X-ray image based on the residual term that has undergone tissue normalization; and
tone mapping and outputting the reconstructed X-ray image.

9. The image processing method of claim 8, wherein the X-ray image is segmented into a background region, an anatomy region, and a collimation region, wherein the anatomy region includes a tissue candidate region, and the residual term is segmented to extract tissue region data corresponding to the tissue candidate region.

10. The image processing method of claim 9, wherein analyzing the original X-ray image comprises extracting the tissue candidate region from the anatomy region using a method that determines the tissue candidate region based on regression to predict a 3D linear plane from a predetermined number of tissue candidate data samples.

11. The image processing method of claim 10, further comprising predicting a tissue model plane based on the extracted tissue region data.

12. The image processing method of claim 11, further comprising generating a background map for the background region.

13. The image processing method of claim 12, further comprising normalizing the residual term based on the predicted tissue model plane information.

14. The image processing method of claim 13, wherein performing the multi-scale transform comprises:

using a Gaussian-Laplacian pyramid structure; generating edge maps for each layer corresponding to high-frequency components; and
correlating each layer corresponding to the high-frequency components and determining whether to suppress or the suppression strength for the edge maps of each layer based on tissue normalization-based weighting values.

15. An image processing system comprising:

an image acquisition device configured to acquire an X-ray image of a human body; a display; and
an image processing device including:

an image analysis module configured to analyze an original X-ray image;
a transformation module configured to perform a multi-scale transform on the original X-ray image based on the result of the analysis and to separate the image into frequency components;

a tissue processing module configured to extract and normalize tissue region data based on a residual term corresponding to a low-frequency band X-ray image among the separated X-ray images;

an inverse transformation module configured to reconstruct the X-ray image based on the residual term that has undergone tissue normalization; and

a control module configured to perform tone mapping and output control of the reconstructed X-ray image.

# FIG. 1

<u>1</u>

# FIG. 2

120                                                   180

# FIG. 3

160

# FIG. 4

Image Analysis (S101)

↓

Log Transform (S103)

↓

Multiscale Transform (S105)

↓

Contrast Control (S107)

↓

Residual Tissue Extraction (S109)

↓

Tissue Plane Estimation (S111)

↓

Background Map Generation (S113)

↓

Tissue Normalization (S115)

↓

Strength Control (S117)

↓

Multiscale Inverse Transform (S119)

↓

Top Mapping (S121)

# FIG. 5

(a)

(b)

# FIG. 6

☐ Body region
▨ Flat region of Body inside
▨ Background region (direct exposure region)

610

(AND)

620    625

630

(a)                    (b)                    (c)

Z depth
value

640

⬭ Flat region (no edge)
● Tissue candidate

Y position

(d)

# FIG. 7

(a)

(b)

Depth z

width x

height y

710

720

| Data count | x1 | x2 | y |
|---|---|---|---|
| 1 | 5 | 3 | 1000 |
| 2 | 4 | 10 | 23000 |
| 3 | 300 | 22 | 12023 |
| ... | ... | ... | ... |
| N | 100 | 232 | 28320 |

(c)

# FIG. 8

(a)

(b)

# FIG. 9

(a)

(b)

(c)

(d)

# FIG. 10

(a)

(b)

FIG. 11

# FIG. 12

# FIG. 13

FIG. 14

FIG. 15

# FIG. 16

Legend: org low freq. signal　Estimated depth plane　Normalized low freq. signal

Tissue plane

(a)　　(b)

FIG. 17

org low freq. signal    Estimated depth plane    Normalized low freq. signal

Tissue plane

(a)                                      (b)

EP 4 631 432 A1

# FIG. 18

EP 4 631 432 A1

org low freq. signal    Estimated depth plane    Normalized low freq. signal

(a)      (b)

Tissue plane

# FIG. 19

org low freq. signal    Estimated depth plane    Normalized low freq. signal

(a)

(b)

Tissue plane

EP 4 631 432 A1

**EP 4 631 432 A1**

## INTERNATIONAL SEARCH REPORT

| | International application No. |
|---|---|
| | **PCT/KR2022/020131** |

| A. | CLASSIFICATION OF SUBJECT MATTER |
|---|---|

**A61B 6/00**(2006.01)i; **G06T 11/00**(2006.01)i

According to International Patent Classification (IPC) or to both national classification and IPC

| B. | FIELDS SEARCHED |
|---|---|

Minimum documentation searched (classification system followed by classification symbols)

A61B 6/00(2006.01); G06Q 50/24(2012.01); G06T 1/00(2006.01); H04N 1/41(2006.01); H04N 5/325(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 엑스레이 (x-ray), 저주파 (low frequency), 레지듀얼 텀 (residual term), 티슈 (tissue), 맵핑 (mapping)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | JP 10-013740 A (GE YOKOGAWA MEDICAL SYST LTD.) 16 January 1998 (1998-01-16)<br>See paragraph [0030]; claim 4; and figures 1 and 2. | 1,8,15 |
| A | | 2-7,9-14 |
| Y | KR 10-2007-0028878 A (KONKUK UNIVERSITY INDUSTRIAL COOPERATION CORP.) 13 March 2007 (2007-03-13)<br>See claim 2. | 1,8,15 |
| A | KR 10-1685823 B1 (INHA UNIVERSITY RESEARCH AND BUSINESS FOUNDATION) 21 December 2016 (2016-12-21)<br>See entire document. | 1-15 |
| A | JP 2002-209113 A (CANON INC.) 26 July 2002 (2002-07-26)<br>See entire document. | 1-15 |

☑ Further documents are listed in the continuation of Box C.　　☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **04 September 2023** | **05 September 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
| --- |
| **PCT/KR2022/020131** |

### C. DOCUMENTS CONSIDERED TO BE RELEVANT

| Category* | Citation of document, with indication, where appropriate, of the relevant passages | Relevant to claim No. |
| --- | --- | --- |
| A | KR 10-2013-0142654 A (SAMSUNG ELECTRONICS CO., LTD.) 30 December 2013 (2013-12-30)<br>See entire document. | 1-15 |

Form PCT/ISA/210 (second sheet) (July 2022)

**INTERNATIONAL SEARCH REPORT**
Information on patent family members

| International application No. |
| --- |
| **PCT/KR2022/020131** |

| Patent document cited in search report | | | Publication date (day/month/year) | Patent family member(s) | | | Publication date (day/month/year) |
| --- | --- | --- | --- | --- | --- | --- | --- |
| JP | 10-013740 | A | 16 January 1998 | None | | | |
| KR | 10-2007-0028878 | A | 13 March 2007 | KR | 10-0719350 | B1 | 17 May 2007 |
| KR | 10-1685823 | B1 | 21 December 2016 | None | | | |
| JP | 2002-209113 | A | 26 July 2002 | JP | 2002-092588 | A | 29 March 2002 |
| | | | | JP | 4428868 | B2 | 10 March 2010 |
| | | | | US | 2002-0031247 | A1 | 14 March 2002 |
| | | | | US | 6975753 | B2 | 13 December 2005 |
| KR | 10-2013-0142654 | A | 30 December 2013 | CN | 103505228 | A | 15 January 2014 |
| | | | | CN | 103505228 | B | 14 June 2019 |
| | | | | CN | 106659409 | A | 10 May 2017 |
| | | | | CN | 106659409 | B | 18 October 2019 |
| | | | | CN | 106659900 | A | 10 May 2017 |
| | | | | CN | 106659900 | B | 17 December 2019 |
| | | | | EP | 2676608 | A1 | 25 December 2013 |
| | | | | EP | 2676608 | B1 | 29 August 2018 |
| | | | | EP | 3182882 | A2 | 28 June 2017 |
| | | | | EP | 3182882 | B1 | 20 May 2020 |
| | | | | EP | 3182883 | A1 | 28 June 2017 |
| | | | | EP | 3182883 | B1 | 23 June 2021 |
| | | | | JP | 2017-525474 | A | 07 September 2017 |
| | | | | JP | 2017-529902 | A | 12 October 2017 |
| | | | | JP | 6487033 | B2 | 20 March 2019 |
| | | | | KR | 10-1870856 | B1 | 25 June 2018 |
| | | | | US | 10016143 | B2 | 10 July 2018 |
| | | | | US | 2013-0343521 | A1 | 26 December 2013 |
| | | | | US | 2015-0342550 | A1 | 03 December 2015 |
| | | | | US | 2016-0045131 | A1 | 18 February 2016 |
| | | | | US | 2016-0045132 | A1 | 18 February 2016 |
| | | | | US | 2016-0045136 | A1 | 18 February 2016 |
| | | | | US | 2016-0367829 | A1 | 22 December 2016 |
| | | | | US | 2017-0209063 | A1 | 27 July 2017 |
| | | | | US | 9125617 | B2 | 08 September 2015 |
| | | | | US | 9451892 | B2 | 27 September 2016 |
| | | | | US | 9451893 | B2 | 27 September 2016 |
| | | | | US | 9629565 | B2 | 25 April 2017 |
| | | | | US | 9895071 | B2 | 20 February 2018 |
| | | | | US | 9993213 | B2 | 12 June 2018 |
| | | | | WO | 2016-028609 | A1 | 25 February 2016 |
| | | | | WO | 2016-028610 | A2 | 25 February 2016 |
| | | | | WO | 2016-028610 | A3 | 02 June 2016 |
| | | | | WO | 2016-028611 | A1 | 25 February 2016 |

Form PCT/ISA/210 (patent family annex) (July 2022)